# FASCICULE DE BREVET EUROPEEN

(11) **EP 2 095 762 B1**
(45) Date de publication et mention de la délivrance du brevet: **11.05.2011**
(21) Numéro de dépôt: 08290179.4
(22) Date de dépôt: 26.02.2008
(51) Int. Cl.: A61B 5/00

(54) **Dispositif d'investigation micro-invasif in vivo comprenant un guide métallique**
Vorrichtung zur mikroinvasiven In-vivo-Untersuchung, die einen metallischen Leiter umfasst
Device for in vivo micro-invasive investigation comprising a metal guide

(43) Date de publication de la demande: 02.09.2009
(73) Titulaire: Biostems Ltd., Dublin (IE)
(72) Inventeur: Pompidou, Alain, 100 Bruxelles (BE); Benhamou, Albert-Claude, 75014 Paris (FR)
(74) Mandataire: Novagraaf Technologies

(56) Documents cités:
- EP-A2- 0 234 928
- EP-B1- 1 358 481
- US-A- 5 938 595
- US-A1- 2005 153 309
- US-A1- 2007 287 991
- US-B2- 7 291 497

## Description

### Domaine technique

La présente invention concerne la fonctionnalisation d'un dispositif d'investigation transpariétal, et l'utilisation d'un tel dispositif pour la fabrication d'un outil destiné au diagnostic d'un cancer, d'une infection, d'une inflammation, ou d'un rejet de greffe chez un patient.

### Etat de la technique

Il est connu dans l'art antérieur des dispositifs d'investigation ou de traitement *in vivo.* De tels dispositifs prennent la forme d'un tube rigide de type endoscope ou d'un cathéter constitué d'un tube flexible qui est inséré dans l'organisme, notamment par les voies naturelles ou les vaisseaux, et qui permet d'atteindre un organe ou un tissu spécifique. Ces dispositifs permettent notamment l'élimination de caillots de sang ou, lorsqu'ils sont associés à des fibres optiques, la visualisation et le contrôle *in vivo* de l'état d'un système, comme le tube digestif, ou d'un organe, comme le colon. Le traumatisme pour le patient résultant de l'utilisation de tels dispositifs est alors minimisé mais reste encore à améliorer. Toutefois, il n'est pas toujours possible d'effectuer une analyse des organes ou des tissus chez un patient avec ces dispositifs. Une telle impossibilité peut résulter de l'accessibilité réduite dudit tissu ou organe au regard de la circulation sanguine ou des voies naturelles, ou alors de la difficulté à effectuer un diagnostic fiable sans recourir à une étude fine des cellules dudit organe ou tissu. Dans ces cas, il est utilisé de façon courante dans l'art antérieur et à ce jour, le prélèvement d'un fragment dudit tissu ou organe (biopsies) afin de contrôler *ex vivo* la morphologie de ces tissus ou organes, ou plus finement celle des cellules qui les constituent, notamment à l'aide d'aiguilles fines (FNA ou « Fine Needle Aspiration» : Engelstein et al., Br. J. Urol., 7 : 210-213, 1994 ; Rodrigues et al., J. Am. Acad. Dermatol., 42 : 735-740, 2000 ; Ariga et al., Am. J. Surg., 184 : 410-413, 2002 ; Pérez-Guillermo et al., Diagn. Cytopathol., 32 : 315-320, 2005 ; Fernandez-Esparrach et al., Arch. Bronconeomol., 43 : 219-224, 2007). En outre, il est également possible d'analyser le statut d'expression d'un certain nombre de marqueurs dont l'expression est corrélée à un état pathologique spécifique (cancer, inflammation ou infection notamment).

Toutefois, ces différentes méthodes, du fait du prélèvement d'une biopsie ou d'aspiration de cellules *in situ,* font subir un traumatisme parfois important audit tissu ou organe et, par voie de conséquence, au patient. L'organisme de ce dernier peut ainsi être fortement éprouvé du fait d'hémorragies ou encore de la cicatrisation consécutive au prélèvement, en particulier pour certains organes (cerveau, pancréas, foie ou poumon).

Il existe donc encore aujourd'hui un besoin pour l'identification de nouvelles méthodologies d'investigations permettant d'effectuer un diagnostic fiable et précis, notamment au regard de l'expression de marqueurs associés spécifiquement à différentes pathologie comme notamment, le cancer, une inflammation ou une infection, tout en limitant les traumatismes infligés au patient.

Le document US 2007/287991 décrit un détecteur pour mesurer le taux d'un marqueur de la douleur. Ce détecteur comprend une aire de détection qui doit être placée dans la région du disque intervertébral, en contact avec les marqueurs de la douleur. Dans le cas où le marqueur de la douleur est un marqueur protéinique, que l'aire de détection comprend une fibre optique. Cette fibre optique est enveloppée d'un revêtement, fait d'un matériau capable de changer de volume en réponse à la présence d'un marqueur de la douleur. Les anticorps sont conjugués à cette enveloppe, et la liaison d'un antigène à l'anticorps provoque la diminution de volume de l'enveloppe. Le revêtement est lui-même enveloppé dans un matériau poreux. Ces trois couches de matériaux constituant l'aire de détection font que le diamètre du détecteur est trop élevé et qu'il n'est donc adapté à être correctement dirigé jusqu'à son site cible sans altérer simultanément les voies de circulation sanguine.

Le document US 2005/153309 décrit une sonde biologique de surveillance pour la détection des maladies, destinée à être introduite dans le sang, et comprenant un corps allongé et une surface liée au corps allongé portant un partenaire de liaison destiné à lier une cible complémentaire. Le partenaire de liaison forme un relief sur la surface de la sonde. Cette sonde est destinée à être introduite dans le sang ou les fluides biologiques, afin de capturer des cellules circulantes ou les protéines ou peptides circulant dans la circulation sanguine ou dans les cavités corporelles. Cette sonde accède au site cible via un accès déjà formé. Toutefois, cette sonde n'est pas perforante, et n'est pas adaptée à être manoeuvré correctement jusqu'à son site cible.

Le document US 7,291,497 décrit un dispositif d'analyse comprenant un système d'investigation constitué d'un guide métalliques dont une des extrémités comporte, sur sa face interne, une série de microplaques sur desquelles sont fixés des réactifs capables de se lier à des analytes et permettant la détection de marqueurs d'une pathologie. Ce système permet la détection d'analytes présents dans les fluides biologiques. Toutefois, sa structure ainsi que son diamètre ne lui permettent pas de pénétrer dans les vaisseaux sanguins, les cellules ou les tissus.

Les fluides sont aspirés dans ce dispositif médical de via l'aiguille, et les analytes sont mis en contact avec les microplaques d'analyse à l'intérieur des microcanaux.
Ainsi, les microplaques sont situées sur la face interne des microcanaux, en amont du moyen de prélèvement constitué de l'aiguille (« microneedle »).
Ce dispositif ne permet pas de mettre en contact direct, sans nécessité d'aspiration, les puits contenant les réactifs et les analytes, les tissus, fluides ou cellules, quelle que soit leur position dans l'organisme.

Le document EP 1 358 481 décrit un dispositif d'analyse comprenant un micro-système d'investigation d'un substrat, une tige souple à une extrémité de laquelle est fixée le micro-système et un système de dilacération, et dont l'autre extrémité est destinée à manoeuvrer le système. Ce micro-système d'investigation comprend, en relief sur la surface de la tige, au moins une série de régions prédéfinies contenant chacune différentes substances chimiques ou biologiques d'investigation ou de traitement. Ce dispositif ne permet pas d'atteindre un organe visé pour y effectuer un micro-prélèvement nécessaire au diagnostic in situ d'une pathologie.

Le document US 5,938,595 décrit une fibre optique sur laquelle est déposé un revêtement pour le diagnostic et le traitement des attaques, permettant un navigation endo-vasculaire uniquement.

Le document EP 234 928 décrit une fibre optique, c'est-à-dire un système de navigation en verre, comprenant des puits où sont disposés des groupements réactifs. Cette fibre optique permet uniquement une navigation dans les vaisseaux, et ne permet pas d'atteindre un organe visé pour y effectuer un micro-prélèvement nécessaire au diagnostic in situ d'une pathologie.

Le brevet EP 1 358 481 décrit un dispositif d'analyse ou de traitement *in vivo* comprenant (i) un micro-système d'investigation d'un substrat autre que par analyse d'un signal fluorescent, (ii) une tige souple à une extrémité de laquelle est fixé ledit microsystème et dont l'autre extrémité est destinée à la manoeuvre dudit micro-système, (iii) un instrument médical possédant une lumière interne dans laquelle ladite tige souple peut coulisser, (iv) un système coulissant de protection du micro-système amovible au niveau du substrat, et (v) au niveau dudit micro-système, un système de dilacération de tissu ou de cellule, éventuellement associé à un ou plusieurs dispositifs choisis parmi un dispositif de surveillance à distance par des récepteurs sensoriels (tactiles, optiques, physico-chimiques et notamment électroniques ou informatiques numérisés), de réalisation de biopsie, de traitement, d'injection locale de produits biologiques ou chimiques. Ce dispositif, lorsqu'il est dirigé jusqu'à son site cible, provoque simultanément une altération des voies de circulation sanguine.

Pour permettre l'effraction de vaisseaux sanguins et la dilacération de tissu ou de cellules, le micro-système est alors associé à un autre système plus rigide assurant cette fonction, de préférence en position distale.

Toutefois, dans le cas de dispositif d'investigation in vivo, le dispositif d'investigation est le plus souvent dirigé vers l'organe ou le tissu cible en utilisant la voie endovasculaire ou endocavitaire. Le couplage du micro-système avec un système de dilacération augmente alors de façon non négligeable le diamètre à l'extrémité du dispositif décrit. Ce dernier se révèle alors être d'un usage complexe pour être correctement dirigé jusqu'à son site cible sans altérer simultanément les voies de circulation sanguine. Simultanément, le couplage de multiples éléments nuisent à la flexibilité d'ensemble du dispositif, et donc à son guidage correct, ainsi qu'à l'obtention de la rigidité nécessaire pour permettre la perforation d'un organe ou d'un tissu.

### Description de l'invention

À la suite d'importantes recherches, l'inventeur a maintenant réussi à développer un dispositif comprenant un guide métallique à une extrémité perforante duquel sont directement couplés des groupements réactifs, notamment des anticorps ou des fragments d'anticorps, spécifiques d'un substrat à tester, et dont l'autre extrémité est destinée à la manoeuvre dudit guide du site d'insertion jusqu'au site de micro-analyse et/ou de micro-prélèvement dudit substrat. Ledit guide peut être inséré dans un système de protection amovible, par exemple un cathéter souple, permettant ainsi de protéger l'extrémité fonctionnalisée dudit guide jusqu'au site de micro-analyse et/ou de micro-prélèvement du substrat à tester constitutif du tissu, de l'organe, ou des cellules de ceux-ci.

Il s'agit, conformément à la présente invention, de structurer la surface du guide dans le but de définir des endroits sur le guide, des puits, où les groupements réactifs seront déposés et où les interactions biochimiques auront lieu. Lesdits « puits » peuvent être réalisés par différents procédés comme par exemple par lithographie par faisceau d'ions focalisés (FIB ou pour « Focused Ion beam » : Xie et al., Nuclear Instruments & Methods in Physics research Section B-beam Interactions with Materials and Atoms, 211(3) : 363-368, 2003), par lithographie par laser suivie par une attaque électrochimique et une ablation laser.

Alternativement, la structuration du guide métallique peut être réalisée dans le but de définir des endroits sur ledit guide, par exemple au moins un sillon dans lequel est solidairement associée au moins une biopuce miniaturisée linéaire, circulaire ou en ruban, où les groupements réactifs seront déposés et où les réactions biochimiques auront lieu.

Ledit dispositif peut en outre être inséré dans un instrument médical possédant une lumière interne dans laquelle ledit guide métallique peut coulisser, et notamment dans une aiguille de ponction transpariétale, notamment transcutanée ou transmuqueuse, ou un endoscope, y compris un système de navigation endovasculaire.

Ledit dispositif peut en outre être associé à une fibre optique, ou le guide métallique dudit dispositif peut être remplacé par une fibre optique à une extrémité perforante de laquelle est associée une bague métallique sur laquelle sont directement couplés des groupements réactifs, notamment des anticorps ou des fragments d'anticorps, spécifiques d'un substrat à tester, permettant ainsi une visualisation fine *in situ*, en vue de la capture *in vivo* des éléments nécessaires au diagnostic et éventuellement à l'évaluation pronostique. Les propriétés de la fibre optique sont utilisées à des fins d'imagerie de repérage et de mise en place du dispositif à partir de la visualisation *in situ.*

Le dispositif selon l'invention du fait de sa simplification présente, tout en restant micro-invasif, une flexibilité ou élasticité améliorée par rapport aux dispositifs de l'art antérieur. Ce dernier peut alors être utilisé bien plus efficacement pour une investigation par voie endocavitaire, notamment par voie endovasculaire, ou par voie transpariétale, notamment pas voie transcutanée ou transmuqeuse. De surcroît, le dispositif selon l'invention du fait de l'utilisation d'un guide métallique, dont une extrémité est couplée à des réactifs spécifiques, présente une rigidité suffisante à son extrémité fonctionnalisée pour perforer efficacement un tissu ou un organe. Enfin, le diamètre du dispositif selon l'invention à son extrémité est suffisamment faible pour permettre une navigation simplifiée dans la circulation sanguine ou dans les cavités naturelles, et surtout minimiser le traumatisme au niveau du tissu ou de l'organe que celui-ci doit perforer.

En outre, et après retrait du dispositif, il est alors possible d'identifier *ex vivo* à l'aide de techniques classiques, comme un dosage immunoenzymatique ou par immunofluorescence, par exemple sur support solide [technique ELISA, puce à protéines (ESPINA et al., J. Immunol. Methods, vol.290, p :121-133, 2004)], la présence et la concentration relative dudit ou desdits substrats spécifiques à l'extrémité du dispositif selon l'invention et donc au niveau du site de micro-analyse et/ou de micro-prélèvement (organe ou tissu).

Enfin, et du fait de la nature métallique du guide, le dispositif présente un signal amélioré en imagerie (artériographie, échographie, scanner, IRM, etc...). Cette propriété permet de simplifier considérablement le radioguidage du dispositif selon l'invention lors de l'intervention jusqu'au tissu ou organe ciblé. Il en est de même pour le couplage dudit dispositif avec une fibre optique à des fins d'imagerie de repérage, et qui permet la visualisation fine *in situ.*

En conséquence, un premier objet de l'invention est un dispositif pour l'analyse d'un substrat, caractérisé en ce qu'il comprend un système d'investigation micro-invasif et/ou de micro-prélèvement dudit substrat, ledit système étant constitué d'au moins un guide métallique à une extrémité Ea duquel est pourvue au moins une série de puits auxquels est directement couplé au moins un groupement réactif spécifique dudit substrat, ladite extrémité fonctionnalisée Ea étant perforante, et dont l'autre extrémité Em est destinée à la manoeuvre dudit guide métallique et éventuellement associée à un système d'aspiration.

Le dispositif selon l'invention, du fait du faible traumatisme qu'il engendre chez le patient, permet de réaliser plusieurs micro-analyses ou micro-prélèvements chez un patient à intervalles réguliers (par exemple, analyse et/ou prélèvements étagés dans le cadre de la prostate). Lesdites analyses et/ou prélèvements successifs permettent ainsi, outre le diagnostic, de suivre l'évolution d'un cancer, d'une inflammation, d'une infection, ou la bonne prise d'une greffe d'organe chez un patient.

Avantageusement, l'extrémité fonctionnalisée Ea peut présenter sur une longueur d'environ 0,5 à 2 cm, au moins une série de 1 à 25 puits, de préférence 2x25 puits, ayant un diamètre moyen d'environ 30 à 80 µm, de préférence d'environ 40 à 60 µm, et de manière tout à fait préférée d'environ 50 µm, une profondeur d'environ 20 à 30 µm, de préférence de 25 µm, lesdits puits étant espacés les uns des autres d'environ 60 à 120 µm. De préférence, les puits ont une paroi lisse ou rugueuse, une forme ovale ou ronde, avec un fond plat ou concave.

Selon un mode de réalisation particulier du dispositif selon l'invention, ledit dispositif comprend en outre un système de protection amovible au niveau de l'extrémité fonctionnalisée Ea.

Selon encore un mode de réalisation particulier du dispositif selon l'invention, ledit dispositif comprend en outre un instrument médical possédant une lumière interne dans laquelle ledit au moins un guide métallique peut coulisser.

Par « guide métallique », on entend par exemple une tige métallique pleine souple ou une tige métallique creuse rigide, ayant un diamètre allant de 0,2 à 3,5 mm et une longueur allant de 5x10⁻² à 2 m, et pouvant être insérée dans un vaisseau sanguin, une petite cavité ou à travers un organe ou un tissu, afin de pouvoir être dirigé depuis le site d'insertion jusqu'au site de micro-analyse et/ou de micro-prélèvement *in situ.*

En particulier, une tige métallique pleine souple peut être constituée d'une fibre optique à une extrémité Ea de laquelle est associée une bague métallique pourvue d'au moins une série de puits auxquels est directement couplé au moins un groupement réactif spécifique dudit substrat, ladite extrémité fonctionnalisée Ea étant perforante.

Avantageusement, ladite bague métallique a une largeur d'environ 0,5 à 2 cm, et peut présenter au moins une série de 1 à 25 puits, de préférence 2x25 puits, ayant un diamètre moyen d'environ 30 à 80 µm, de préférence d'environ 40 à 60 µm, et de manière tout à fait préférée d'environ 50 µm, une profondeur d'environ 20 à 30 µm, de préférence de 25 µm, lesdits puits étant espacés les uns des autres d'environ 60 à 120 µm. De préférence, les puits ont une paroi lisse ou rugueuse, une forme ovale ou ronde, avec un fond plat ou concave.

Par « guide métallique », on entend en outre une tige pleine souple ou creuse, rigide ou flexible constituée en tout ou partie d'un alliage métallique dont les caractéristiques de flexibilité, de rigidité, d'oxydation et d'immunogénicité sont compatibles avec une telle utilisation chez l'être vivant et en particulier chez l'animal, et tout particulièrement chez l'homme. De tels alliages biocompatibles peuvent être identifiés simplement par l'homme du métier au regard de ses connaissances générales et comprennent notamment les aciers inoxydables, les alliages à base de titane, de nickel, de cobalt, ou des mélanges de ceux-ci.

L'inventeur a pu démontrer qu'un guide à base d'un alliage de titane et de nickel (alliage de Nitinol) présentait des propriétés particulièrement intéressantes en terme de flexibilité d'ensemble et de rigidité à son extrémité pour être utilisé efficacement pour les voies endovasculaires ou endocavitaires et également pour perforer efficacement un tissu ou un organe tout en minimisant le traumatisme (la taille de la perforation au niveau dudit tissu ou organe est par exemple de l'ordre de 0,05 à 0,5 mm², et de préférence de l'ordre de 0,07 mm²).

Avantageusement, le guide métallique est à base d'un alliage de nickel et de titane, de préférence à base de Nitinol (guides métalliques commercialisés par la société Euroflex).

Ledit guide métallique peut être recouvert, à l'exception de l'extrémité fonctionnalisée Ea, d'un polymère hydrophile, de préférence un hydrogel, ou d'une couche polymère protectrice poreuse, ayant une épaisseur allant d'environ 0,1 à 51 µm. Avantageusement, le polymère protecteur est constitué d'un film de parylène, de TiO₂ ou d'OptoDex® (Arrayon Biotechnology, Suisse), et plus préférentiellement d'un film de parylène.

Le système de protection amovible dans lequel est inséré le guide métallique peut prendre de multiples formes, et notamment celle d'un cathéter souple, qui peuvent être déterminées simplement par l'homme du métier, par exemple des formes utilisables pour la voie endovasculaire, endocavitaire, transpariétale, et notamment transcutanée.

Ledit système de protection amovible peut être inséré par voie endovasculaire notamment pour atteindre les vaisseaux du coeur, le cerveau, le poumon, le pancréas, le rein, et le foie.

Ledit système de protection amovible peut être inséré par des accès endocavitaires, notamment par le biais d'un endoscope, par voie orale, anale, urogénitale et respiratoire, ou ORL par voie transmuqueuse, ou encore par voie transcutanée par le biais d'une ponction au niveau de la peau, pour atteindre par exemple la glande mammaire, et notamment jusqu'au rein ou dans une articulation ou en transpariétal dans le foie, le poumon ou le rein, mais également par voie transmuqueuse, notamment pour le tube digestif.

L'inventeur a ainsi mis en évidence que le dispositif selon l'invention permet alors d'atteindre des tissus ou organes, normalement difficiles d'accès, par les voies endovasculaire, endocavitaire ou transpariétale, et notamment transmuqueuse ou transcutanée, utilisées classiquement.

Plus généralement, l'inventeur a mis en évidence que le dispositif selon l'invention permet du fait de ses caractéristiques spécifiques de flexibilité d'ensemble et de rigidité à son extrémité d'atteindre et de perforer par voie transpariétale (transcutanée, transmuqueuse), endovasculaire ou endocavitaire certains organes et tissus appartenant au système digestif de l'oropharynx au rectum (y compris foie et pancréas), au système urogénital (dont vessie, rein, prostate, testicule, ovaire et glande mammaire), au système trachéo-bronchique (dont poumon), au système ORL (dont oreille et rhinopharynx), au système ostéo-articulaire (dont cavités synoviales), au système endocrinien, au système neurocérébral ou au système tégumentaire, rendant ainsi possible la réalisation de diagnostics sur des pathologies qui nécessitaient jusqu'alors la réalisation de biopsies voir des ponctions profondes agressives comme les biopsies trans-hépatiques, y compris le prélèvement de cellules par aspiration à l'aide d'aiguilles fines (technique dite de « fine needle aspiration » ou FNA).

Avantageusement, ledit système de protection amovible dans lequel est inséré le guide métallique prend la forme d'un cathéter souple adapté pour la voie endovasculaire ou endocavitaire.

Le dispositif selon l'invention est alors particulièrement adapté pour effectuer une investigation, par exemple, au niveau des artères et des veines, des vaisseaux du coeur, de la prostate, de la glande mammaire, du pancréas, du rein, du muscle cardiaque, du système nerveux central et ses cavités ou canaux, du cerveau ou du foie.

Selon un premier mode de réalisation particulier du dispositif selon l'invention, le système de protection amovible dans lequel est inséré le guide métallique est lui-même inséré dans un endoscope. Le dispositif selon l'invention est alors particulièrement adapté pour une administration par la voie endocavitaire.

Le dispositif selon l'invention est alors particulièrement adapté pour effectuer une investigation, par exemple, au niveau du système trachéo-bronchique (dont poumon), du système digestif du pharynx au rectum (y compris foie et pancréas), du système urogénital (dont vessie, rein, prostate, testicule, ovaire et glande mammaire), du système ophtalmique (canaux lacrymaux), du système ORL (dont oreille et rhinopharynx), du système ostéo-articulaire, ou au niveau du cerveau ou de la glande mammaire par la voie endogalactophorique.

Selon un second mode de réalisation particulier du dispositif selon l'invention, le guide métallique constitué d'une aiguille fine de ponction transpariétale, et notamment de ponction transcutanée ou transmuqueuse, peut être inséré dans un système de protection amovible, par exemple un cathéter souple. Le dispositif selon l'invention est alors particulièrement adapté pour une administration spécifiquement par la voie transcutanée ou transmuqueuse.

Le dispositif selon l'invention est alors particulièrement adapté pour effectuer une investigation, par exemple, au niveau des téguments (peau, cuir chevelu, etc...), du sein, du rein, du poumon, du foie, du muscle, de l'appareil ostéo-musculaire ou ostéo-articulaire, ou des glandes endocrines (notamment thyroïde, parathyroïde, surrénales, testicules, glandes mammaires ou ovaires).

En ce qui concerne l'extrémité Ea fonctionnalisée du dispositif selon l'invention, elle est pourvue d'au moins une série de puits auxquels sont directement couplés des groupements réactifs spécifiques d'un substrat à tester.

Par « groupements réactifs spécifiques », on entend par exemple une séquence d'acide nucléique [ADN (amplifiat, fragment de gène, EST, SNP) ou ARN] complémentaire d'une séquence d'acide nucléique à détecter, un antigène spécifique d'un anticorps à détecter ou un anticorps ou un fragment d'anticorps spécifique d'un antigène à détecter, de préférence un anticorps ou un fragment d'anticorps.

Lesdits groupements réactifs spécifiques sont disposés dans les micro-puits de l'extrémité fonctionnalisée du dispositif selon l'invention selon une gamme croissante ou décroissante. L'homme du métier peut déterminer simplement, à l'aide de ses connaissances générales et d'expériences de routine, ladite gamme en fonction de l'affinité du groupement réactif pour son substrat. À titre d'exemple, la gamme du groupement réactif est de l'ordre de 50 à 500 µg/ml, de préférence de 10 à 100 µg/ml, pour un réactif, notamment un anticorps, ayant une affinité pour son substrat, notamment un antigène, de l'ordre de 10⁻⁹.

Par « anticorps », on entend, de préférence une immunoglobuline de mammifère, notamment humaine, et de manière particulièrement préférée une IgG.

Par « fragments d'anticorps », on entend des fragments d'anticorps capables de maintenir une fixation spécifique de leur antigène. À titre d'exemple de tels fragments d'anticorps, on peut citer les fragments Fab, Fab', F(ab')₂ ou Fv.

Des méthodes de couplage de réactifs, notamment de protéines sur un support métallique sont bien connues de l'homme du métier. De telles méthodes, du fait de la faible réactivité chimique des acides aminés, nécessitent en général l'activation de la surface métallique soit par des mécanismes d'oxydation, soit en recouvrant cette dernière d'au moins une couche de molécules de liaisons, le plus souvent de polymères (celle-ci peut présenter par exemple des groupements thiols, acides carboxyliques et/ou des amines). À titre d'exemple de telles méthodes, on peut citer l'adsorption sur des supports métalliques de molécules fonctionnelles s'organisant en monocouches auto-assemblées (self-assembled monolayer SAMs) et notamment les alcanethiols (voir notamment WITTSTOCK and SCHUHMANN, Anal. Chem., vol.69, p :5059-5066, 1997 ; et la demande internationale WO 03/006948) ou le pyrrole (par polymérisation électrochimique de pyrrole biotinylé; DUPONT-FILLIARD et al., Anal. Chim. Acta., vol.449, p :45-50, 2001).

Par couplage de l'anticorps ou du fragment d'anticorps à cette couche de molécules fonctionnelles, on entend une liaison covalente (comme des ponts disulfures entre les groupements thiols libres des alcanethiols) ou non covalente (comme une liaison streptavidine-biotine entre la biotine d'une couche de polymères d'un complexe pyrolle/biotine et la streptavidine d'un complexe streptavidine/anticorps ou fragment d'anticorps) streptavidine).

Selon un premier mode de réalisation préféré, le dispositif selon l'invention comprend au moins un guide métallique dont une extrémité Ea est couplée avec au moins un groupement réactif, de préférence un anticorps ou un fragment d'anticorps, spécifique d'un marqueur (antigène) d'un cancer, notamment du cancer du sein, de l'ovaire, de la prostate, du colon, intra-abdominal, du rein, du cerveau, du foie, du poumon, du pancréas ou d'une glande endocrine (notamment thyroïde, testicules ou ovaires).

À titre d'exemple de marqueur du cancer du sein, on peut citer le marqueur CA 15-3 (Carcinoma-Associated Antigen 15-3 ; Duffy MJ, Shering S, Sherry F, McDermott E, O'Higgins N, Int J Biol Markers, 2000 Oct-Dec ;15(4) : 330-3), CA 27-29 (Carcinoma-Associated Antigen 27-29 ; Kaohsiung J, J Med Sci, 1999 Sep ; 15(9) : 520-8), CEA (Carcinoembryonic antigen ; Soletormos G, Nielsen D, Schioler V, Mouridsen H, Dombernowsky P, Eur J Cancer, 2004 Mar ;40 (4) : 481-6) ; TPA (Tissue Polypeptide Antigen) ; TPS (Tissue Polypeptide Specific Antigen; Given M, Scott M, Mc Grath JP, Given HF, Breast, 2000 Oct; 9 (5) : 277-80), HER2 (Fehm T, Jager W, Kramer S, Sohn C, Solomayer E, Wallwiener D, Gebauer G., Anticancer Research, 2004 May-Jun ; 24 (3b) : 1987-92), ER (Estrogene Receptor ; Platet N, Cathiard AM, Gleizes M, Garcia M., Crit Rev Oncol Hematol, 2004 Jul ; 51 (1) : 55-67), PR (Progesterone Receptor ; Duffy MJ, Clin Chem, 2005 Mar ;51(3) : 494-503. Epub 2005 Jan 6.), Ki-67 (cell proliferation-associated antigen of antibody Ki-67 ; Schlüter C, Duchrow M, Wohlenberg C, Becker MH, Key G, Flad HD, Gerdes J, J Cell Biol, 1993 Nov ; 123(3) : 513-22) et UPA (Urokinase Plasmogen Activator ; Duffy MJ, Crit Rev Clin Lab Sci, 2001 Jun ;38(3) : 225-62).

À titre d'exemple de marqueur du cancer de l'ovaire, on peut citer le marqueur CA125 (Carcinom Antigen 125; Moss EL, Hollingworth J, Reynolds TM, J Clin Pathol, 2005 Mar ; 58(3) : 308-12), CA 15-3 et CEA (Valenzuela P, Mateos S, Tello E, Lopez-Bueno MJ, Garrido N, Gaspar MJ, Eur J Gyn Oncol, 2003 ; 24(1) : 60-2).

À titre d'exemple de marqueur du cancer de la prostate, on peut citer le marqueur PSA (Prostate-Specific Antigen; Gray MA, *Clin Lab.,2005 ;* 51(3-4) :127-33) ; PMSA (Prostate-Specific Membrane Antigen) et AR (Androgen Receptor ; Birtle AJ, Freeman A, Masters JR, Payne HA, Harland SJ, BJU Int, 2005 Aug ; 96(3) : 303-7).

À titre d'exemple de marqueur du cancer du colon, on peut citer le marqueur CEA (Duffy MJ, Clin Chem, 2001 Apr ; 47(4) : 624-30), CA 19-9 (Carcinom Antigen 19-9), CA242 (Carcinom Antigen 242), CA 72-4 (Carcinom Antigen 72-4), TPA, TPS (Duffy MJ, van Dalen A, Haglund C, Hansson L, Klapdor R, Lamerz R, Nilsson O, Sturgeon C, Topolcan O, Eur J Cancer, 2003 Apr ; 39 (6) : 718-27*) .*

À titre d'exemple de marqueur du cancer intraabdominal, on peut citer le marqueur CEA ou CA 19-9 (Coban E, Samur M, Bozcuk H, Ozdogan M, Int J Biol Markers, 2003 Jul-Sep ; 18(3) : 177-81).

À titre d'exemple de marqueur du cancer du pancréas, on peut citer le marqueur TA90-IC (a 90-kDa immugenic Tumor-associated Antigen), CA-19-9 (Chung MH, Gupta RK, Bilchik AJ, Ye W, Yee R, Morton DL, Curr Surg,2002 March-April ;59(2) : 194-198), TPS, HCG beta (hCG beta , Human Chorionic Gonadotropin beta), CA 72-4, CEA, CA 19-9, CA 242 (Louhimo J, Alfthan H, Stenman UH, Haglund C, Oncology, 2004 ; 66(2) : 126-31).

À titre d'exemple de marqueur du cancer du foie, on peut citer le marqueur alpha-foetoprotéine.

À titre d'exemple de marqueur du cancer du poumon, on peut citer le marqueur Cyfra A41 (Cytokeratin fragment 41), SCC (Squamous Cell Carcinoma antigen), ACE (Angiotensin Converting Enzyme), CA 19-9, CA 125, NSE (Neuron Specific Enolase), chromogranine A, CYFRA 21-1 (Cytokeratin fragment 21-1) CA 15-3.

Selon un second mode de réalisation préféré, le dispositif selon l'invention comprend au moins un guide métallique dont une extrémité Ea est couplée avec au moins un réactif, de préférence un anticorps ou un fragment d'anticorps, spécifique d'un marqueur spécifique d'une inflammation, notamment de l'arthrite rhumatoïde.

À titre d'exemple de marqueur de l'arthrite rhumatoïde, on peut citer notamment l'IL-1β, l'IL-1Rα, l'IL-2, l'IL-2R, l'IL-4, l'IL-5, l'IL-6, l'IL7, l'IL8, l'IL10, l'IL12p40P70, l'IL-13, l'IL-15, l'IL-17, le TNFα, l'IFNα, l'IFNγ, le GM-CSF, le MIP-1, l'IP-10, le MIG, l'Eotaxine, le RANTES et le MCP-1 (COCKRUM et al., Lab Automation, BTi, October 2005, p :19-21*).*

Selon un troisième mode de réalisation préféré, le dispositif selon l'invention comprend au moins un guide métallique dont une extrémité Ea est couplée avec au moins un réactif, de préférence un anticorps ou un fragment d'anticorps, spécifique d'un marqueur spécifique d'une infection, notamment d'une infection virale, bactérienne ou parasitaire.

De nombreux marqueurs infectieux sont connus de l'homme du métier et ce dernier pourra identifier sans difficulté le ou les marqueurs spécifiques associés à une infection donnée.

Selon un quatrième mode de réalisation préféré, le dispositif selon l'invention comprend au moins un guide métallique dont une extrémité Ea est couplée avec au moins un réactif, de préférence un anticorps ou un fragment d'anticorps, spécifique d'un marqueur spécifique du rejet de greffe.

De nombreux marqueurs du rejet de greffe sont connus de l'homme du métier. À titre d'exemple, on peut citer MIP-1β et la VE-cadhérine pour la greffe du coeur (ROUSSOULIÈRES et al., Circulation, vol.111(20), p :2636-2644, 2005).

Au regard des marqueurs spécifiques qui sont décrits précédemment, l'homme du métier au regard de ses connaissances générales pourra identifier facilement et sans expérimentation excessives les anticorps ou fragments d'anticorps spécifiques pouvant être utilisés dans le dispositif selon l'invention. À titre d'exemple, de tels anticorps, on peut citer les anticorps disponibles chez TEBU ou AXXORA. L'homme du métier pourra également obtenir de tels anticorps par des méthodes d'immunisation bien connues.

De même, l'homme du métier pourra identifier sans difficulté les acides nucléiques spécifiques adaptés pouvant être utilisés dans le dispositif selon l'invention.

Avantageusement, le dispositif selon l'invention, tout au moins en sa partie terminale fonctionnalisée en contact avec le substrat à analyser, présente un niveau d'assurance de stérilité (SAL pour Sterility Assurance Level) de l'ordre de 10⁻⁶. Différentes alternatives sont envisageables afin d'atteindre ce niveau de stérilité. Une possibilité est de stériliser le dispositif en l'absence des groupements réactifs spécifiques du substrat à détecter puis d'ajouter ces derniers en conditions stériles. Une autre possibilité est de stériliser le dispositif après l'ajout des groupements réactifs, ce qui nécessite d'utiliser des techniques de stérilisation ne réduisant pas significativement l'activité desdits groupements réactifs (par exemple stérilisation par l'oxyde d'éthylène ou radiation).

Un procédé de détection *ex-vivo* d'un substrat présent dans un tissu ou organe comprend les étapes suivantes :
a) l'incubation de l'extrémité fonctionnalisée Ea d'un dispositif selon l'invention, avec une solution comprenant au moins un agent de détection spécifique dudit substrat, après que ladite extrémité ait été mise en contact avec ledit tissu ou organe devant être examiné.
b) la détection dudit substrat.

L'étape d'incubation est effectuée pendant un temps suffisant pour que l'agent de détection en solution, notamment un anticorps, puisse se fixer spécifiquement au substrat (marqueur, antigène, anticorps, etc...), notamment u antigène, éventuellement présent à l'extrémité du dispositif. L'homme du métier peut déterminer simplement à l'aide de ses connaissances générales et d'expériences de routine, ce temps d'incubation en fonction de l'affinité de l'agent de détection en solution, notamment un anticorps, pour son substrat, notamment un antigène. Ce temps d'incubation est également fonction de la température de la solution lors de cette incubation. À titre d'exemple, le temps d'incubation est de l'ordre de 1 minute à 2 heures, de préférence de 5 minutes à 1 heure, et de manière particulièrement préférée de 10 à 30 minutes, pour une température comprise entre 20°C (température ambiante) et 37°C.

Avantageusement, l'agent de détection en solution est différent du réactif spécifique couplé à l'extrémité fonctionnalisée du dispositif selon l'invention.

De préférence, l'agent de détection est un anticorps.

Avantageusement, l'anticorps en solution et l'anticorps couplé à l'extrémité fonctionnalisée du dispositif selon l'invention sont chacun un anticorps polyclonal, de préférence lesdits anticorps sont identiques.

Avantageusement, l'anticorps en solution et l'anticorps couplé à l'extrémité fonctionnalisée du dispositif selon l'invention sont chacun un anticorps monoclonal, de préférence lesdits anticorps sont différents.

Avantageusement, l'anticorps en solution est marqué, et notamment est couplé à une enzyme, par exemple à la peroxydase ou à la phosphatase alcaline.

Selon un premier mode de réalisation particulier du procédé, le procédé comprend en outre une étape a') d'incubation de ladite extrémité dans une solution comprenant au moins un agent de détection spécifique de l'agent de détection de l'étape a), intercalée entre les étapes a) et b).

L'homme du métier pourra identifier simplement et à l'aide de ses connaissances générales, les anticorps adaptés au procédé selon l'invention. À titre d'exemple, il est possible d'utiliser dans cette deuxième étape un anticorps reconnaissant spécifiquement des immunoglobulines de souris, si de telles immunoglobulines de souris dirigées spécifiquement contre le substrat (marqueur, antigène, anticorps, etc...) à identifier sont utilisés à l'étape a).

Selon un second mode de réalisation particulier du procédé, le procédé comprend une étape de lavage à la suite de l'étape d'incubation a), et éventuellement de l'étape a'), laquelle étape de lavage permet d'éliminer les anticorps qui ne se sont pas fixés spécifiquement au marqueur (l'antigène).

Le protocole d'une telle étape de lavage fait là encore partie des connaissances générales ou peut être déterminé simplement par des expériences de routine. À titre d'exemple, une telle étape est effectuée avec une solution comprenant une concentration plus ou moins élevée de détergent (de 0,05 à 1%), comme le TRITON X100® ou le TWEEN 20®, et ceci en fonction de l'affinité de l'anticorps en solution pour son antigène spécifique.

L'étape de détection est effectuée en mettant en évidence une activité, notamment enzymatique, couplée à l'anticorps utilisé à l'étape a) ou, éventuellement à l'étape a').

Le protocole utilisé pour cette étape de détection est fonction du marqueur utilisé, et notamment de l'enzyme utilisée, par exemple la peroxydase et la phosphatase alcaline, et fait partie des connaissances générales de l'homme du métier.

Cette étape de détection permet de déduire la quantité de substrat spécifique (par exemple d'antigène) fixé à l'extrémité fonctionnalisée du dispositif et finalement la quantité de substrat spécifique présent au niveau de l'organe ou du tissu où a été effectué(e) la micro-analyse et/ou le micro-prélèvement.

Finalement, les différents groupements réactifs utilisables pour réaliser le procédé sont bien connus de l'homme du métier et incluent, notamment, les réactifs utilisés pour la technique de dosage immunoenzymatique ou par immunofluorescence, par exemple sur support solide [technique ELISA, puce à protéines (ESPINA et al., précité, 2004)].

Un deuxième objet de l'invention consiste en l'utilisation d'un dispositif selon l'invention, pour la fabrication d'un outil destiné au diagnostic d'un cancer, d'une inflammation, d'une infection ou d'un rejet de greffe chez un patient.

Selon un mode de réalisation particulier de l'invention, ledit outil de diagnostic peut comprendre au moins un guide métallique inséré dans un cathéter souple inséré dans un endoscope.

Selon un autre mode de réalisation particulier de l'invention, ledit outil de diagnostic peut comprendre au moins un guide métallique consistant en une aiguille de ponction transpariétale, et notamment de ponction transcutanée ou transmuqueuse, pouvant être inséré dans un système de protection amovible, par exemple un cathéter souple. En outre, le système de protection amovible et le guide métallique coopèrent de manière à permettre la mise en contact de l'extrémité Ea fonctionnalisée dudit guide avec le site de micro-analyse et/ou de micro-prélèvement.

Dans ces deux modes de réalisation particuliers de l'invention, ledit au moins un guide métallique peut être associé sur au moins une partie de sa longueur à une fibre optique en vue de repérage et de positionnement.

Avantageusement, ledit outil de diagnostic est administré par voie endocavitaire.

Ledit outil permet ainsi d'effectuer une micro-analyse et/ou un micro-prélèvement au niveau du système digestif du pharynx au rectum (y compris foie et pancréas), du système urogénital (dont vessie, urètre, rein, prostate), du système trachéo-bronchique (dont poumon), du système ORL (dont oreille et rhinopharynx), du système ostéo-articulaire (dont cavités synoviales).

De manière préférée, ledit outil de diagnostic est administré par voie transpariétale, notamment par voie transmuqueuse ou transcutanée.

Un tel outil de diagnostic permet ainsi d'analyser des tissus ou organes difficiles à atteindre par la voie endocavitaire ou endovasculaire utilisée habituellement. Un tel outil de diagnostic permet ainsi d'effectuer une micro-analyse et/ou un micro-prélèvement par voie transpariétale au niveau de la peau, des testicules, de la prostate, de l'ovaire ou des glandes mammaires, mais également du rein ou du foie, du système nerveux (notamment du cerveau) ainsi que du système endocrinien (par exemple la thyroïde).

Les exemples qui suivent permettent d'illustrer l'invention et sont donnés à titre non-limitatif.

### Brève description des Figures

- La Figure 1 représente différentes possibilités pour la structuration des guides métalliques.
- La Figure 2 représente des photographies de microscopie électronique à balayage de différents trous réalisés par FIB (la grande rugosité de surface due aux inhomogénéités de gravure peut être observée).
- La Figure 3 représente une explication et une observation de l'effet d'ombrage de la technique de fraisage assistée par la fluorine.
- La Figure 4 représente la suite des opérations de microfaçonnage.
- La Figure 5 représente (a) des trous hémisphériques observés par microscope optique (b) des séries de trous observés sur un guide à base de Nitinol par microscopie électronique à balayage (c) détail d'une cavité (d) comparaison de la rugosité de surface dans la cavité avec la rugosité de la surface du guide.
- La Figure 6 représente des photos de microscopie électronique à balayage montrant le même trou hémisphérique que dans la Figure 5 après le traitement de polissage électrochimique.
- La Figure 7 représente un schéma du dispositif de l'invention pour l'immunocapture avec un permier anticorps monoclonal (AcM 1) et la révélation de l'antigène ACE avec un second anticorps monoclonal (AcM 2).
- La Figure 8 représente les résultats d'ELISA avec l'anticorps de capture 5910 et la révélation avec l'anticorps 5909 (absorbance obtenue avec un sérum positif en antigène ACE)
- La Figure 9 représente les résultats d'ELISA avec l'anticorps de capture 5910 et la révélation avec l'anticorps 5909 (absorbance obtenue avec un sérum négatif en antigène ACE)
- La Figure 10 représente les résultats d'ELISA avec l'anticorps de capture 5905 et la révélation avec l'anticorps 5909 (absorbance obtenue avec un sérum positif en antigène ACE)
- La Figure 11 représente les résultats d'ELISA avec l'anticorps de capture 5905 et la révélation avec l'anticorps 5909 (absorbance obtenue avec un sérum négatif en antigène ACE)
- La Figure 12 représente les résultats d'ELISA des tiges plastiques rigides avec l'anticorps de capture 5910 et la révélation avec l'anticorps 5909 (absorbance obtenue avec un sérum positif en antigène ACE)

Ces figures sont des illustrations de la sensibilité et de la spécificité de la méthode utilisée.

### EXEMPLE 1 PREPARATION D'UN GUIDE METALLIQUE A BASE DE NITINOL ET SON ACTIVATION

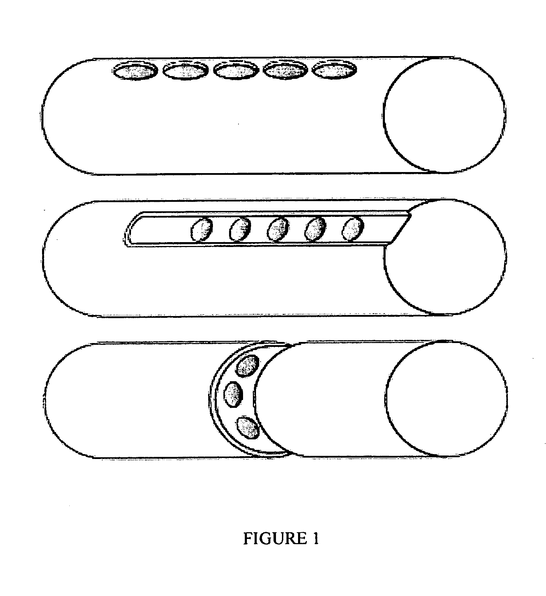

La surface d'un guide métallique à base de Nitinol (Euroflex) est structurée dans le but de définir des endroits, par exemple des puits, où les groupements réactifs seront déposés et où les interactions biochimiques auront lieu (Figure 1).

Lesdits « puits » peuvent être réalisés par différents procédés comme par exemple par lithographie par faisceau d'ions focalisés (FIB ou pour « Focused Ion beam » : Xie et al., Nuclear Instruments & Methods in Physics research Section B-beam Interactions with Materials and Atoms, 211(3) : 363-368, 2003), par lithographie par laser suivie par une gravure électrochimique et une ablation laser.

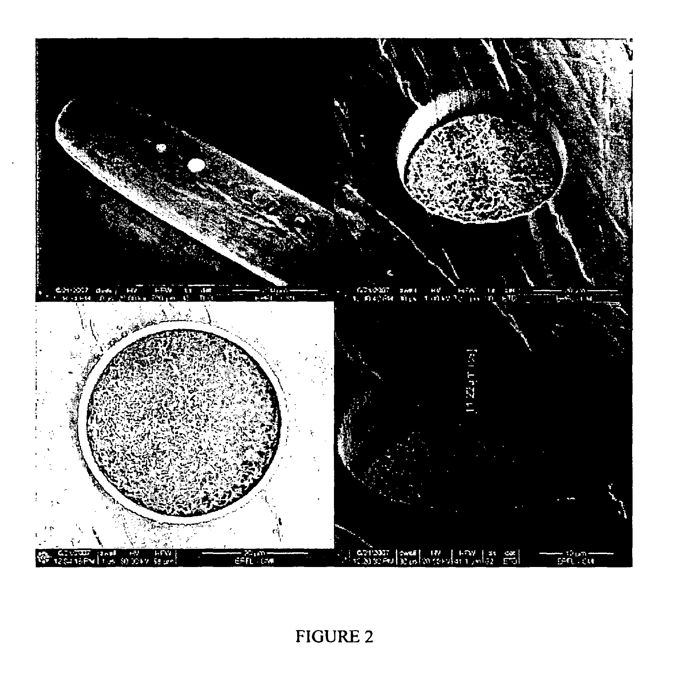

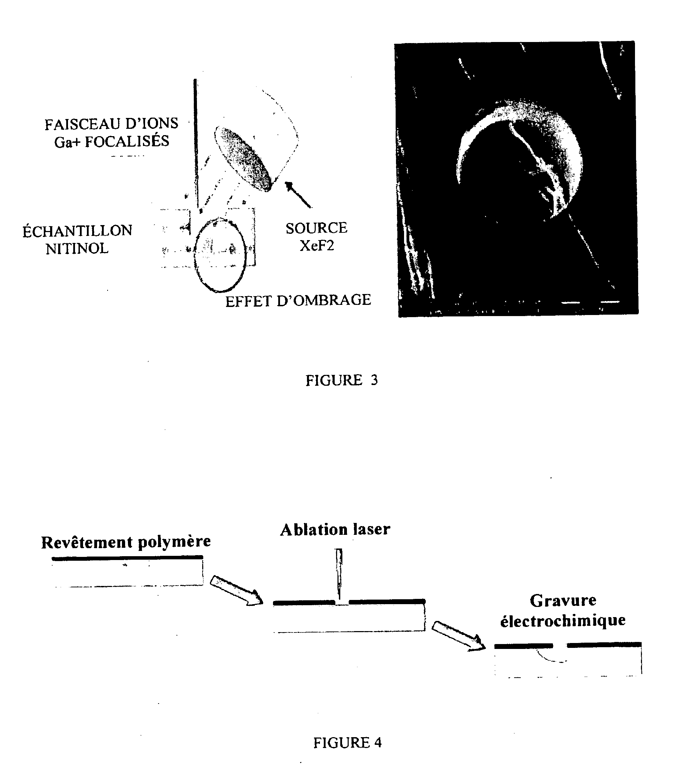

Avec la technique FIB, la machine crée un faisceau d'ions, qui est focalisé sur la surface qui doit être structurée. Sous l'action mécanique du faisceau d'ions, les atomes du matériau de surface sont éliminés de la surface. Des trous d'un diamètre de 20 µm peuvent être formés avec la technique FIB dans un délai raisonnable avec un facteur de morsure de 8 µm³s⁻¹ sous un courant de faisceau de 20 nA. La Figure 2 montre des trous de diamètres de 5, 20 et 40 µm avec une profondeur de 10 et 20 µm. La surface du fond du trou est rugueuse du fait du re-dépôt du matériel pulvérisé au cours de l'attaque. Le facteur de morsure a été mesuré à 200 nm min⁻¹ sur une aire circulaire de 40 µm de diamètre et avec un courant de faisceau de 20 nA. Cela donne un facteur de morsure de 0,2 µm³ nC⁻¹ (environ 5 µm³s⁻¹), qui correspond à un temps de procédure de 20 min pour effectuer un trou de 20 µm de diamètre et de 20 µm de profondeur. Afin d'améliorer la rugosité de la surface, une technique de fraisage assistée par fluorine (XeF₂) a été utilisée ; une très faible rugosité de surface a alors été obtenue, mais comme la source de XeF₂ n'était pas exactement dans l'axe du faisceau de gravure, un effet d'ombrage a été observé (Figure 3).

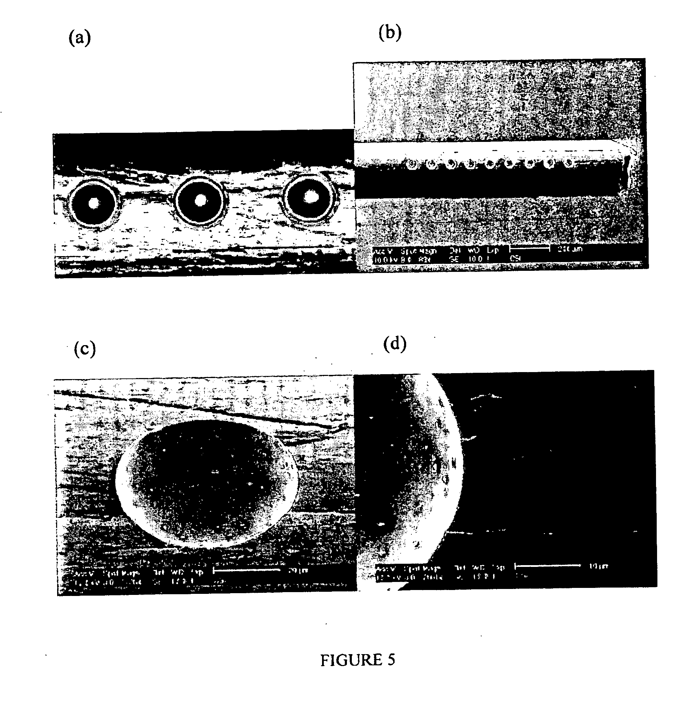

La technique de lithographie par laser et de gravure électrochimique consiste dans une première étape à recouvrir la surface avec une couche de polymère. Dans une seconde étape, la couche polymère est façonnée en utilisant l'ablation laser. Dans une troisième étape, la surface est gravée en utilisant une gravure électrochimique isotrope à travers l'ouverture faite dans la couche polymère (Figure 4). La Figure 5 présente les résultats de différents tests de structuration sur des guides métalliques à base de Nitinol.

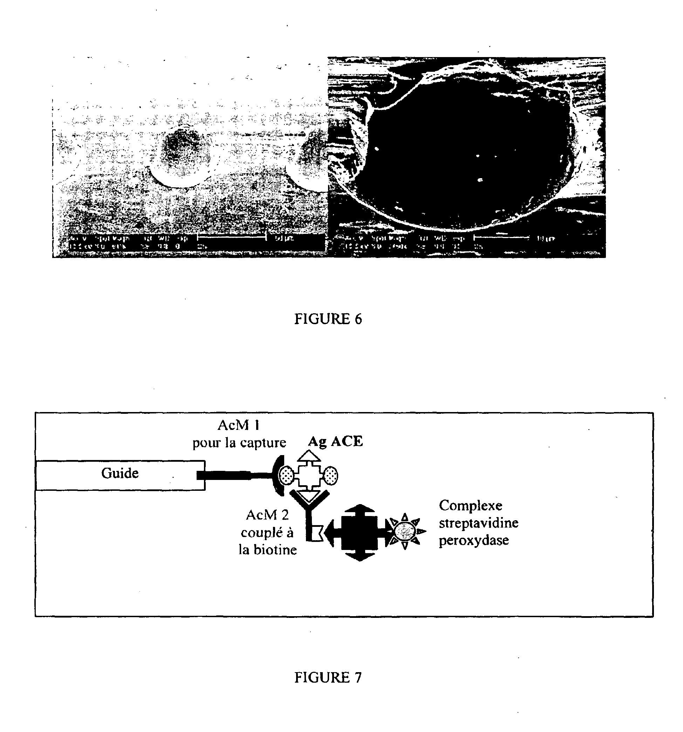

Par ailleurs, les guides métalliques à base de Nitinol qui sont utilisés *in vivo* sont habituellement traités par polissage électrochimique, qui remplace la couche d'oxyde natif NiTi avec une couche TiO₂ biocompatible. Les guides façonnés avec des trous sont soumis à ce procédé afin d'évaluer l'influence du procédé sur la structure des trous (Figure 6).

Une autre façon de préparer les cavités sur les surfaces des guides à base de Nitinol recourt à l'ablation laser. L'utilisation de courtes impulsions laser permet l'évaporation locale du métal sans affecter le métal environnant du fait de la chaleur générée. Les plus petites dimensions rapportées sont de l'ordre de 20 µm.

Si les trois procédés décrits ci-dessus permettent la réalisation de puits, c'est le procédé par gravure électrochimique qui donne les meilleurs résultats.

### EXEMPLE 2 TRAUMATISME CONSECUTIF A L'INSERTION IN VIVO D'UN GUIDE METALLIQUE DANS UN ORGANE PARTICULIER

Pour ces expériences, des micro-guides métalliques (MTI 0,012" Silver speed) ont été utilisés, lesquels guides métalliques étaient insérés dans des micro-cathéters.

Le dispositif a été introduit chez des porcs, sous anesthésie générale, au niveau d'une ponction puis au niveau du scarpa jusqu'au rein par la voie endovasculaire (via l'artère fémorale). Ce guidage a été assuré par le suivi dudit dispositif dans l'artère fémorale par artériographie.

Une fois positionné à l'entrée du rein, le dispositif a été introduit dans le rein par effraction endoartérielle. Cette pénétration dans le tissu s'est faite à une profondeur de quelques millimètres et ledit dispositif y a été maintenu pendant une dizaine de minutes.

Finalement, le dispositif a alors été retiré.

Les animaux ont alors été euthanasiés et les reins de ces derniers ont été prélevés pour évaluer l'état de ceux-ci après pénétration du dispositif selon l'invention.

Les résultats ont montré qu'aucune hémorragie importante du rein n'était associée à l'effraction. La lésion la plus importante constatée présentait une dimension de 3 x 1 mm au niveau du site d'effraction.

Le dispositif selon l'invention permet donc d'accéder à un organe tout en étant très faiblement invasif.

### EXEMPLE 3 MICRO-TRAUMATISME CONSECUTIF A L'INSERTION DU DISPOSITIF AU NIVEAU DU FOIE

Des micro-guides métalliques (MTI 0,012" Silver speed) ont été utilisés, lesquels micro-guides métalliques étaient placés dans un fibroscope à la différence de l'exemple 1.

Le dispositif a été introduit chez des porcs, sous anesthésie générale, au niveau d'une ponction au niveau du scarpa puis jusqu'au foie par navigation endoartérielle (via l'artère fémorale). Ce guidage a été assuré par le suivi dudit dispositif dans l'artère fémorale par artériographie.

Une fois positionné à proximité du foie, le dispositif a été introduit dans celui-ci. Cette pénétration dans le tissu s'est faite à une profondeur de quelques millimètres et ledit dispositif y a été maintenu là encore pendant une dizaine de minutes.

Finalement, le dispositif a alors été retiré.

Comme précédemment, le prélèvement du foie après l'opération a permis de juger de l'agressivité de l'intervention sur l'organe.

Aucune lésion macroscopiquement visible n'a été observée à la surface du foie. A la coupe, la présence de deux foyers hémorragiques intra-parenchymateux de siège sous-capsulaire de 1,5x0,4 cm et de 1,8x0,5 cm ont été observés. Histologiquement, l'architecture hépatique est en tout point conservée avec une congestion des sinusoïdes, des veinules portes et des veines centrolobulaires sans aucune autre anomalie notable.

### Conclusion

Les résultats ont montré que les lésions hémorragiques sont minimes : deux lésions macroscopiques mineures ont pu être observées sans aucune destruction des cellules parenchymateuses et avec une simple congestion des capillaires et des veines centro-lobulaires.

L'utilisation d'un guide métallique permet donc d'obtenir un traumatisme mineur et, dans tous les cas, largement inférieur à celui résultant d'une biopsie.

### EXEMPLE 4 ETUDE DES PARAMETRES POUR LA CONCEPTION ET LA REALISATION D'UN DISPOSITIF PERMETTANT L'IMMUNOCAPTURE ET LA DETECTION DE L'ANTIGENE ACE IN VITRO SUR SUPPORTS SOLIDES

Le dispositif utilise le principe de la technique ELISA permettant de mettre en évidence l'antigène ACE. Deux anticorps monoclonaux reconnaissant sur cet antigène des épitopes différents, ont été utilisés pour la capture (AcM1) et la révélation de l'antigène ACE (AcM2). Ces anticorps monoclonaux ayant le même isotype (IgGl), la révélation de l'antigène ACE a été réalisée à l'aide d'un anticorps monoclonal couplé à la biotine et d'un complexe streptavidne-peroxydase (Figure 7).

Deux types de support ont été utilisés, soit des plaques pour ELISA, soit des tiges plastiques rigides.

### Plaques pour ELISA (Greiger)

100 µl d'un anticorps monoclonal dirigé contre l'antigène ACE (clone 5910 ou clone 5905, produits chez la souris et commercialisés par Medix Biochemical) dilué (1/5000 au 1/128000) en tampon carbonate/bicarbonate ont été déposés par puits, et la plaque a été placée pendant 1 heure à 37°C. UN témoin négatif a été réalisée en remplaçant l'anticorps par du tampon carbonate/bicarbonate.

Après trois lavages avec 250 µl par puits de PBS les sites libres de la plaque ont été saturés par 200 ml de PBS-BSA (sérum albumine bovine) 3% pendant 2 heures à 37°C.

Les puits ont ensuite été lavés trois fois par 250 µl de PBS-Tween à 0,5% avant l'ajout de 100 µl par puits d'un sérum positif en antigène ACE dilué au 1/10, 1/100, 1/1000 en PBS-Tween, et la plaque a été incubée pendant 1 heure à 37°C.

Trois lavages de 250 µl par puits ont été effectués en PBS-Tween avant l'addition de 100 µl par puits d'un anticorps monoclonal dirigé contre l'antigène ACE (clone 5909 produit chez la souris et commercialisés par Medix Biochemical, qui diffère des précédents anticorps de capture utilisés par sa constante d'affinité et par les épitopes reconnus) biotinylé au 1/500 en PBS-Tween, et la plaque a été de nouveau incubée pendant 1 heure à 37°C.

Après trois lavages au PBS-Tween, 100 µl de conjugué streptavidine couplé à la peroxydase dilué au 1/2000 ont été ajoutés dans chaque puits et incubés pendant 1 heure à 37°C.

Après trois lavages au PBS-Tween, la révélation a été effectuée par addition de 200 µl par puits du mélange substrat (H₂O₂) et chromogène (OPD, Sigma) en tampon citrate-phosphate (pH 5).

En parallèle, la même opération a été réalisée en utilisant comme antigène, un sérum de patient « normal » (témoin négatif avec un dosage d'ACE <5 UI/ml).

La réaction a ensuite été stoppée par ajout de 50 µl d'acide sulfurique à 1M par puits. L'absorbance a été lue à 492 nm sur un lecteur de plaques (réf : ELX.800 UV).

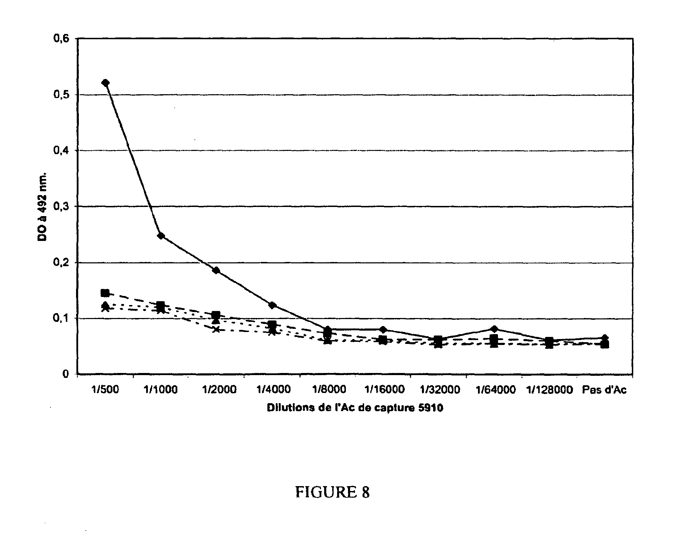

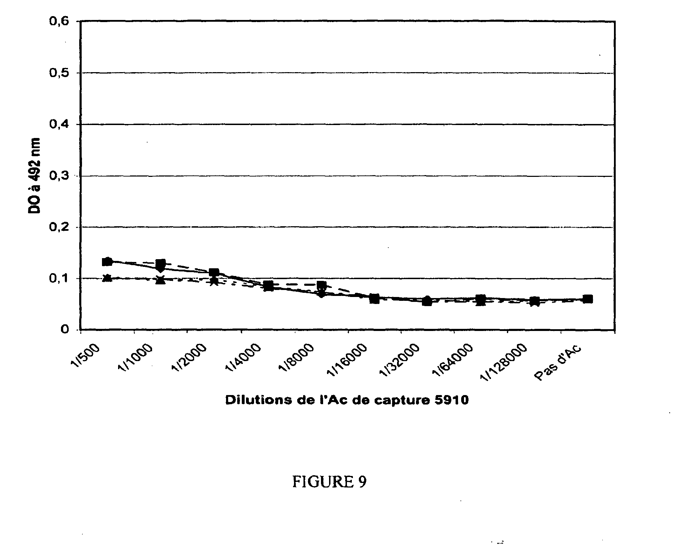

Les résultats obtenus en utilisant l'anticorps monoclonal 5910 pour l'immunocapture (dilué au 1/500 puis de demi en demi jusqu'au 1/128000) et la révélation par l'anticorps monoclonal 5909 biotinylé sont présentés dans la Figure 8 pour le sérum positif en antigène ACE, et dans la Figure 9 pour le sérum négatif en ACE.

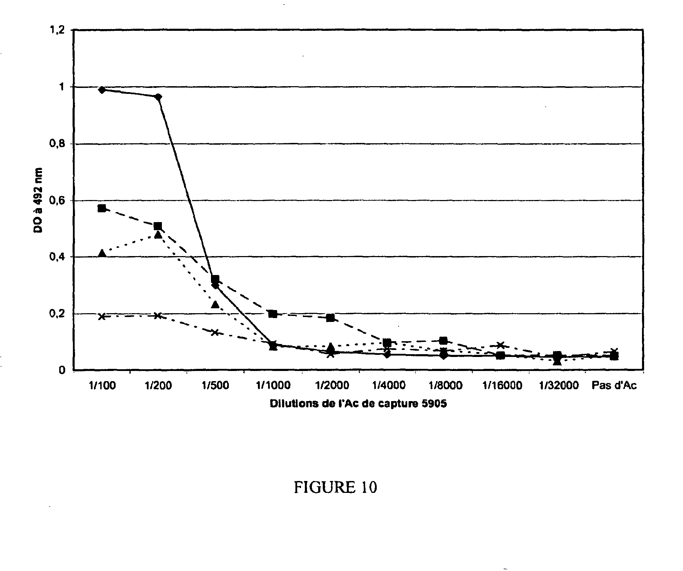

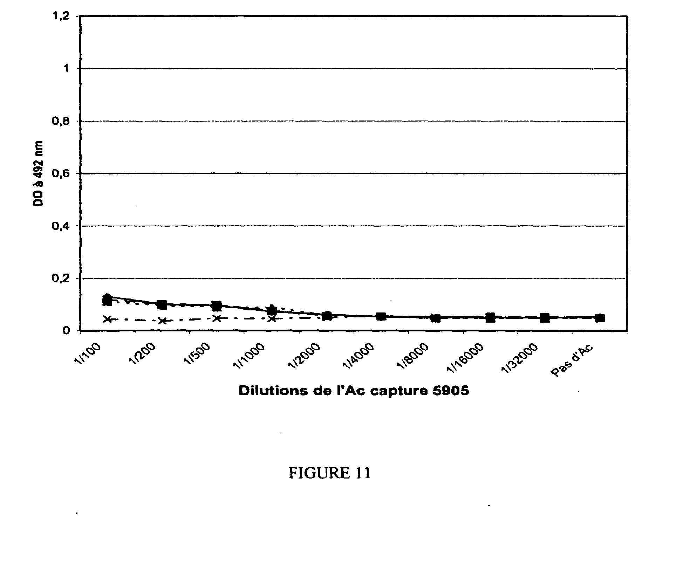

Les résultats obtenus en utilisant l'anticorps monoclonal 5905 pour l'immunocapture (dilué au 1/100, 1/200, 1/500 puis de demi en demi jusqu'au 1/32000) et la révélation par l'anticorps monoclonal 5909 biotinylé sont présentés dans la Figure 10 pour le sérum positif en antigène ACE, et dans la Figure 11 pour le sérum négatif en ACE.

Légendes des Figures 8 à 11 :
Ordonnée : absorbance (DO) à 492 nm
Abscisse : dilutions de l'anticorps de capture (5910 ou 5905)
◆ = dilution sérum positif en ACE au 1/10
■ = dilution sérum positif en ACE au 1/100
Δ = dilution sérum positif en ACE au 1/1000
× = pas de sérum

Les résultats montrent que le sérum positif en antigène ACE au 1/10 donne une absorbance (DO) supérieure à 0,5 lorsque l'anticorps monoclonal de capture est utilisé au 1/500 (Figure 8). Dans les mêmes conditions, le sérum négatif en antigène ACE donne une DO inférieure à 0,15 (Figure 9).

Toutefois, il est à noter que de meilleurs résultats ont été obtenus avec le couple anticorps monoclonal 5905 de capture et anticorps monoclonal 5909 de détection (Figures 10 et 11) qu'avec le couple anticorps monoclonal 5910 de capture et anticorps monoclonal 5909 de détection (Figures 8 et 9). En effet, une DO de 1 a été observée avec le sérum positif en antigène ACE dilué au 1/10 (Figure 10) alors que le sérum négatif en antigène ACE donne dans les mêmes conditions une DO de 0,1 (Figure 11). Ces résultats ont été confirmés en utilisant l'anticorps monoclonal 5910 de capture à différentes dilutions (données non représentées).

### Supports plastiques rigides

Dans une première étape, des supports plastiques rigides sous la forme de tiges de 2 à 3 cm de longueur et de 0,5 à 1 mm de diamètre ont été activés.

Dans une seconde étape, les supports ainsi activés ont été placés dans des microtubes à hémolyse de 1 ml (Fisher) et ont été fonctionnalisés avec un anticorps monoclonal dirigé contre l'antigène ACE (clone 5910 produit chez la souris et commercialisé par Medix Biochemical) et dilué au 1/50, 1/100, 1/250, 1/500 en tampon carbonate/bicarbonate (250 µl/tube) pendant 1 heure à 37°C. Un témoin négatif a été réalisé en remplaçant l'anticorps monoclonal par du tampon carbonate/bicarbonate. Après fixation et lavages, la saturation a été effectuée avec 500 µl de PBS-BSA 3% pendant une nuit à +4°C.

Les supports ont ensuite été incubés avec 250 µl de sérum positif en antigène ACE dilué au 1/10, 1/100 en PBS ou avec du sérum d'un sujet « sain » (témoin négatif en antigène ACE) à la même dilution pendant 1 heure à 37°C.

Un anticorps monoclonal dirigé contre l'antigène ACE (clone 5909 produit chez la souris et commercialisé par Medix Biochemical, qui diffère du clone 5910 par sa constante d'affinité et par les épitopes reconnus) purifié, à 1 mg/ml, a été dialysé une nuit à 4°C contre du tampon borate 0,1 M pH 8,8. Une solution de biotine à 10 mg/ml en DMSO a alors été ajoutée à raison de 50 µg/mg d'anticorps. Après incubation de 4 heures à température ambiante et sous agitation, du chlorure d'ammonium 1 M a été ajouté, à raison de 20 µl/ 250 µg de biotine, et la solution obtenue a été de nouveau incubée durant 1 minute à température ambiante. Après blocage de la réaction, l'anticorps marqué a été dialysé 24 heures à +4°C contre du PBS et cet anticorps marqué a été conservé sous forme d'aliquotes à -20°C.

Après 3 lavages en PBS-Tween, les supports ont été incubés avec 250 µl de l'anticorps 5909 biotinylé et dilué au 1/500 en PBS-Tween pendant 1 heure à 37°C.

La détection de la biotine (ester de l'acide 6-biotinamidocaproylamido-caproïque et de N-hydroxysuccinimide, Sigma) a été mise en évidence à l'aide d'un complexe streptavidine-peroxydase (Amersham Biosciences) dilué au 1/2000 en PBS pendant 1 heure à 37°C.

La révélation de l'activité enzymatique a été réalisée par addition de 750 µl par tube du mélange substrat (H₂O₂) et chromogène (OPD, Sigma) en tampon citrate-phosphate (pH 5).

La réaction a ensuite été stoppée par ajout d'acide sulfurique à 1M. L'absorbance a été lue à 492 nm.

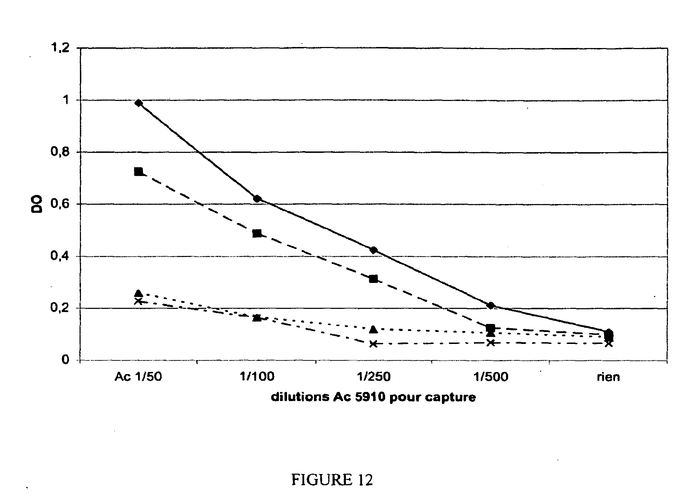

Les résultats d'ELISA sur tiges plastiques sont présentés dans la Figure 12.

Légendes de la Figure 12 :
Ordonnée : absorbance (DO) à 492 nm
Abscisse : dilutions de l'anticorps 5910 de capture
◆ = dilution antigène ACE au 1/10
■ = dilution antigène ACE au 1/100
Δ = dilution témoin négatif au 1/10
× = dilution témoin négatif au 1/100

En général, les résultats montrent que les DO sont 7 à 10 fois plus élevées avec le sérum positif en antigène ACE que celles obtenues avec le sérum négatif en antigène ACE.

Les meilleurs résultats ont été obtenus avec les supports plastiques rigides sur lesquels ont été fixés l'anticorps monoclonal 5910 de capture dilué au 1/50 ou au 1/100.

La concentration en antigène ACE ayant été la mieux détectée, correspond au sérum du patient dilué au 1/100 soit à 6 UI/ml (proche du taux considéré comme « normal » : < 5UI/ml) et lorsque la dilution de l'anticorps monoclonal 5909 de détection est au 1/500.

L'utilisation des supports plastiques permet de valider la spécificité et la sensibilité des processus d'immunocapture sur tige métallique fonctionnalisée selon le protocole décrit précédemment.

### Conclusion

Les bons résultats obtenus pour la détection de l'antigène ACE avec les techniques d'immunocapture et de révélation *in vitro,* valident l'évaluation des dispositifs « tiges fonctionnalisées » permettant la capture *in vivo* de l'antigène ACE suivie d'une révélation *ex vivo.*

### EXEMPLE 5 IDENTIFICATION DE L'EXPRESSION DU MARQUEUR ACE DANS UNE TUMEUR DU SEIN PAR EXEMPLE SOUS CONTROLE DE TECHNIQUES D'IMAGERIE, NOTAMMENT RADIOLOGIQUES

Selon le protocole décrit dans la demande PCT WO 03/006948, dans une première étape, une couche d'alcanethiol est adsorbée sur l'une des extrémités de guides métalliques à base de Nitinol (Euroflex) dans une première étape. Dans une seconde étape, les fonctions thiol libres de cette couche permettent la formation de ponts disulfures avec un anticorps monoclonal dirigé contre l'antigène ACE.

Le guide métallique obtenu est alors introduit dans une aiguille à biopsie adaptée en vue de son utilisation chez l'animal ou chez l'être humain.

Un examen anatomopathologique extemporané est réalisé à l'aide de ce dispositif sur une pièce opératoire (tumeur mammaire), après son retrait chez une patiente souffrant d'un cancer du sein. Alternativement, lorsque les conditions éthiques médicales sont réunies, une micro-incision au niveau du sein est réalisée sous anesthésie locale ou générale chez une patiente souffrant d'un cancer du sein. L'aiguille, dans laquelle est inséré le guide métallique couplé à l'anticorps dirigé contre l'antigène ACE, est introduite dans la tumeur ou par la micro-incision puis dirigée jusqu'à la tumeur en suivant sa progression par imagerie, et notamment par échographie.

Ledit système de guidage micro-invasif permet alors de sortir l'extrémité du guide métallique couplée à l'anticorps dirigé contre l'antigène ACE. L'extrémité du guide métallique est alors introduite dans la tumeur (par perforation) à une profondeur de l'ordre de quelques millimètres. Après un faible temps d'attente, de l'ordre d'une dizaine de minutes, qui permet l'immunocapture de l'antigène ACE éventuellement exprimé par la tumeur, le dispositif est retiré.

Le micro-prélèvement se limite à une immunocapture de l'analyte *in vivo* et ne nécessite pas de biopsie.

Finalement, le dispositif est retiré puis un dosage ELISA du marqueur ACE est réalisé sur l'extrémité du dispositif avec un anticorps monoclonal dirigé contre l'antigène ACE qui se différencie de l'anticorps de capture par sa constante d'affinité vis-à-vis de l'antigène ACE et par les épitopes reconnus, et qui est couplé à la biotine.

La révélation de l'activité enzymatique à l'aide d'un complexe streptavidine-peroxydase permet de conclure à l'expression du marqueur ACE par la tumeur et de moduler en conséquence la thérapie à utiliser pour traiter au mieux la patiente.

### EXEMPLE 6 CANCER CUTANE

Selon le protocole décrit dans la demande PCT WO 03/006948, une couche d'alcanethiol a été adsorbée sur l'extrémité d'un guide métallique à base de Nitinol (Euroflex) dans une première étape. Dans une seconde étape, les fonctions thiols libres de cette couche ont permis la formation de ponts disulfures avec un anticorps monoclonal dirigé contre le marqueur FAP (Fibroblast-activation protein ; RETTIG et al., Proc. Natl. Acad. Sci. USA, vol.85, p :3110, 1988).

Le guide métallique obtenu est alors introduit au niveau d'une tumeur cutanée chez l'animal ou chez l'homme lorsque les conditions éthiques médicales sont réunies, ou encore au niveau d'une tumeur cutanée chez un patient souffrant d'un cancer de la peau après son ablation pour un examen anatomopathologique classique ou extemporané.

Le micro-prélèvement se limite à une immunocapture *in vivo* et ne nécessite là encore pas de biopsie spécifique.

Finalement, le dispositif est retiré et un dosage ELISA du marqueur FAP est réalisé sur l'extrémité du dispositif avec un anticorps monoclonal dirigé contre le marqueur FAP couplé à la peroxydase.

La révélation de l'activité péroxydase permet de conclure à l'expression du marqueur FAP par la tumeur et de moduler en conséquence la thérapie à utiliser pour traiter au mieux le patient.

## Revendications

1. Dispositif d'analyse, comprenant un système d'investigation micro-invasif et/ou de micro-prélèvement d'un substrat, ledit système étant constitué d'au moins un guide métallique comportant :
- une extrémité Ea dudit guide métallique dont la surface est structurée de manière à définir, dans ladite surface, au moins une série de puits auxquels est directement couplé au moins un groupement réactif spécifique dudit substrat, ladite extrémité Ea étant perforante, et
- une autre extrémité Em dudit guide métalliques, destinée à la manoeuvre dudit guide métallique;
le dispositif étant **caractérisé en ce que** ledit groupement réactif est disposé dans lesdits puits.

2. Dispositif selon la revendication 1 comprenant en outre un système de protection amovible au niveau de l'extrémité Ea.

3. Dispositif selon la revendication 2, dans lequel ledit système de protection amovible est un cathéter souple.

4. Dispositif selon l'une quelconque des revendications 1 à 3 comprenant en outre un instrument médical possédant une lumière interne dans laquelle ledit au moins un guide métallique peut coulisser.

5. Dispositif selon la revendication 4, dans lequel ledit instrument médical est choisi dans le groupe comprenant une aiguille de ponction transpariétale, et/ou un endoscope, y compris un système de navigation endovasculaire.

6. Dispositif selon la revendication 5, dans lequel ladite aiguille de ponction transpariétale est une aiguille de ponction transcutanée ou transmuqueuse.

7. Dispositif selon l'une quelconque des revendications 1 à 6, dans lequel ledit au moins un guide métallique est choisi dans le groupe comprenant une ou plusieurs tiges métalliques pleines souples et/ou une ou plusieurs tiges métalliques creuses rigides, ayant un diamètre allant de 0,3 à 3,5 mm et une longueur allant de 5x10⁻² à 2 m.

8. Dispositif selon l'une quelconque des revendications 1 à 7, dans lequel ledit au moins un guide métallique est associé sur au moins une partie de sa longueur à un système de visualisation.

9. Dispositif selon la revendication 8, dans lequel le système de visualisation est une fibre optique.

10. Dispositif selon l'une quelconque des revendications 1 à 9, dans lequel ledit au moins un guide métallique est constitué en tout ou partie d'un alliage métallique choisi dans le groupe comprenant des aciers inoxydables, des alliages à base de titane, de nickel, de cobalt, ou d'un mélange de ceux-ci.

11. Dispositif selon la revendication 10, dans lequel ledit guide métallique est constitué en tout ou partie d'un alliage de titane et de nickel.

12. Dispositif selon l'une quelconque des revendications 1 à 11, dans lequel ledit au moins un guide métallique est recouvert, à l'exception de l'extrémité Ea, d'une couche polymère protectrice ayant une épaisseur allant de 2x10⁻³ à 1 µm.

13. Dispositif selon l'une quelconque des revendications 1 à 12, dans lequel ledit au moins un groupement réactif est spécifique d'un substrat ou antigène spécifique du cancer, d'une inflammation, d'une infection ou du rejet de greffe.

14. Dispositif selon l'une quelconque des revendications 1 à 13, dans lequel ledit au moins un groupement réactif spécifique d'un substrat est un anticorps ou un fragment d'anticorps choisi dans le groupe constitué des fragments Fab, Fab', F(ab')₂ et Fv.

15. Utilisation d'un dispositif selon l'une quelconque des revendications 1 à 14, pour la fabrication d'un outil destiné au diagnostic d'un cancer, d'une inflammation, d'une infection ou d'un rejet de greffe.

16. Utilisation selon la revendication 15, dans laquelle ledit outil comprend au moins un guide métallique inséré dans un cathéter souple inséré dans un endoscope, et est destiné au diagnostic d'un cancer, d'une inflammation, d'une infection ou d'un rejet de greffe.

17. Utilisation selon la revendication 15, dans laquelle ledit outil comprend au moins un guide métallique constitué d'une aiguille de ponction transpariétale et inséré dans un cathéter souple, et est destiné au diagnostic d'un cancer, d'une inflammation, d'une infection ou d'un rejet de greffe.

18. Utilisation selon la revendication 17, dans laquelle ledit au moins un guide métallique et le cathéter souple coopèrent ensemble de manière à mettre en contact l'extrémité fonctionnalisée Ea avec le site de micro-analyse et/ou de micro-prélèvement.

19. Utilisation selon l'une quelconque des revendications 16 à 18, dans laquelle la voie transpariétale est choisie dans le groupe constitué des voies transmuqueuse, et transcutanée.

20. Utilisation selon l'une quelconque des revendications 16 à 19, dans laquelle ledit au moins un guide métallique est associé sur au moins une partie de sa longueur à une fibre optique.

## Claims

1. An analysis device comprising a system for a micro-invasive and/or micro-sampling investigation of a substrate, said system being constituted of at least one metal guide comprising:
- an end Ea of said metal guide, which the surface of which is structured so as to define, in said surface, at least a series of wells to which is directly coupled at least one reactive group specific to said substrate, said end Ea being perforating, and
- another end Em of said metal guide, intended for handling said metal guide
the device being **characterised in that** said reactive group being placed in said wells.

2. A device according to claim 1, further comprising a removable protection system at the level of the end Ea.

3. A device according to claim 2, wherein said removable protection system is a flexible catheter.

4. A device according to any one of claims 1 to 3 further comprising a medical instrument having an internal opening into which said at least one metal guide can slide.

5. A device according to claim 4, wherein said medical instrument is selected in the group comprising a transparietal aspiration needle and/or an endoscope, inclusive of an endovascular navigation system.

6. A device according to claim 5, wherein said transparietal aspiration needle is a transcutaneous or transmucosal aspiration needle.

7. A device according to any one of claims 1 to 6, wherein said at least one metal guide is selected in the group comprising one or several full flexible solid metal rods and/or one or several hollow rigid metal rods having a diameter from 0.3 to 3.5 mm and a length from 5x10⁻² to 2 m.

8. A device according to any one of claims 1 to 7, wherein said at least one metal guide is associated with a visual display system on at least a part of the length thereof.

9. A device according to claim 8, wherein said visual display system is an optical fibre.

10. A device according to any one of claims 1 to 9, wherein said at least one metal guide is constituted, as a whole or in part, of a metal alloy selected in the group comprising stainless steel, titanium-, nickel-, cobalt- based alloys or a mixture thereof.

11. A device according to claim 10, wherein said metal guide is constituted, as a whole or in part, of a titanium and nickel alloy.

12. A device according to any one of claims 1 to 11, wherein said at least one metal guide is coated, except for the end Ea, with a protective polymer layer having a thickness of 2x10⁻³ to 1 µm.

13. A device according to any one of claims 1 to 12, wherein said at least one reactive group is specific to a substrate or an antigen specific of cancer, an inflammation, an infection, or a graft rejection.

14. A device according to any one of claims 1 to 13, wherein said at least one reactive group specific to a substrate is an antibody or a fragment of antibody selected in the group constituted of Fab, Fab', F(ab')₂ and Fv fragments.

15. Use of a device according to any one of claims 1 to 14 for manufacturing a tool intended for diagnosing a cancer, an inflammation, an infection or a graft rejection.

16. The use according to claim 15, wherein said tool comprises at least one metal guide inserted into a flexible catheter inserted into an endoscope, and is intended for diagnosing cancer, an inflammation, an infection or a graft rejection.

17. The use according to claim 15, wherein said tool comprises at least one metal guide constituted of a transparietal aspiration needle and inserted into a flexible catheter and is intended for diagnosing cancer, an inflammation, an infection or a graft rejection.

18. The use according to claim 17, wherein said at least one metal guide and the flexible catheter cooperate together so as to place the functionalized end Ea in contact with the micro-analysis and/or micro-sampling site.

19. The use according to any one of claims 16 to 18, wherein the transparietal route is selected in the group constituted of the transmucosal and transcutaneous routes.

20. The use according to any one of claims 16 to 19, wherein said at least one metal guide is associated with an optical fibre on at least a part of the length thereof.

## Patentansprüche

1. Ein Analysegerät, das ein System zur mikroinvasiven und/oder Mikrosampling-Untersuchung eines Substrats enthält, wobei besagtes System, aus mindestens einer Metallführung besteht, die folgendes enthält:
- ein Ea-Ende der besagten Metallführung, deren Oberfläche so strukturiert ist, dass in besagter Oberfläche mindestens eine Serie von Mulden definiert wird, an die zumindest eine für besagtes Substrat spezifische Reaktionsgruppe direkt gekoppelt ist, wobei besagtes Ea-Ende perforierend ist und
- ein anderes Em-Ende der besagten Metallführung zur Bedienung der besagten Metallführung,
wobei das besagte gerät **dadurch gekennzeichnet ist, dass** besagte Reaktionsgruppe in besagten Mulden platziert wird.

2. Ein Gerät gemäß Anspruch 1, das zudem ein entfernbares Sicherheitssystem im Bereich des Ea-Endes enthält.

3. Ein Gerät gemäß Anspruch 2, in dem besagtes entfernbares Sicherheitssystem aus einem flexiblen Katheder besteht.

4. Ein Gerät gemäß einem beliebigen der Ansprüche 1 bis 3, das zudem ein medizinisches Instrument mit einer internen Öffnung enthält, in der wie gesagt mindestens eine Metallführung gleiten kann.

5. Ein Gerät gemäß Anspruch 4, wobei besagtes medizinisches Instrument innerhalb einer Gruppe gewählt wird, zu der eine transparietale Saugnadel und/oder ein Endoskop, inklusive ein endovaskulares Navigationssystem gehören.

6. Ein Gerät gemäß Anspruch 5, wobei besagte transparietale Saugnadel eine transkutane oder transmukosale Saugnadel ist.

7. Ein Gerät gemäß einem beliebigen der Ansprüche 1 bis 6, wobei wie gesagt mindestens eine Metallführung innerhalb der Gruppe gewählt wird, zu der ein oder mehrere flexible massive Metallstäbe und/oder ein oder mehrere starre hohle Metallstäbe mit einem Durchmesser von 0, bis 3,5 mm und einer Länge von 5x10⁻² bis 2 m gehören.

8. Ein Gerät gemäß einem beliebigen der Ansprüche 1 bis 7, wobei wie gesagt mindestens eine Metallführung mit einem Bildschirmsystem an mindestens einem Teil der Länge hiervon verbunden ist.

9. Ein Gerät gemäß Anspruch 8, wobei besagtes Bildschirmsystem eine optische Faser ist.

10. Ein Gerät gemäß einem beliebigen der Ansprüche 1 bis 9, wobei wie gesagt eine Metallführung ganz oder teilweise aus einer Metalllegierung besteht, die innerhalb der Gruppe gewählt wird, zu der rostfreier Edelstahl, Legierungen auf Titan-, Nickel-, Kobaltbasis oder eine Mischung hiervon gehören.

11. Ein Gerät gemäß Anspruch 10, wobei besagte Metallführung ganz oder teilweise aus einer Titan- und Nickelleggierung besteht.

12. Ein Gerät gemäß einem beliebigen der Ansprüche 1 bis 11, wobei wie gesagt mindestens eine Metallführung mit Ausnahme des Ea-Endes mit einer Polymerschutzschicht beschichtet ist, die eine Dicke von 2x10⁻³ bis 1µm hat.

13. Ein Gerät gemäß einem beliebigen der Ansprüche 1 bis 12, wobei wie gesagt mindestens eine Reaktionsgruppe spezifisch für ein Substrat oder ein für Krebs, eine Entzündung, eine Infektion oder eine Transplantatabstoßung spezifisches Antigen ist.

14. Ein Gerät gemäß einem beliebigen der Ansprüche 1 bis 13, wobei wie gesagt mindestens eine für ein Substrat spezifische Reaktionsgruppe ein Antikörper oder ein Antikörperfragment ist, das innerhalb der Gruppe gewählt wird, zu der die Fragmente Fab, Fab', F(ab')₂ und Fv gehören.

15. Verwendung eines Geräts gemäß einem beliebigen der Ansprüche 1 bis 14 zur Herstellung eines Werkzeugs zur Diagnose von Krebs, einer Entzündung, einer Infektion oder einer Transplantatabstoßung.

16. Verwendung gemäß Anspruch 15, wobei besagtes Werkzeug mindestens eine Metallführung enthält, die in einen flexiblen Katheder eingeführt wird, der in ein Endoskop eingeführt wird und der Diagnose von Krebs, einer Entzündung, einer Infektion oder einer Transplantatabstoßung dient.

17. Verwendung gemäß Anspruch 15, wobei besagtes Werkzeug mindestens eine Metallführung enthält, die aus einer transparietalen Saugnadel besteht und in einen flexiblen Katheder eingeführt wird und der Diagnose von Krebs, einer Entzündung, einer Infektion oder einer Transplantatabstoßung dient.

18. Verwendung gemäß Anspruch 17, wobei wie gesagt mindestens eine Metallführung und ein flexibler Katheder derart zusammenarbeiten, dass das funktionalisierte Ea-Ende in Kontakt mit dem Ort der Mikroanalyse und/oder des Mikrosampling gebracht wird.

19. Verwendung gemäß einem beliebigen der Ansprüche 16 bis 18, wobei der transparietale Weg innerhalb der Gruppe gewählt wird, zu der die transmukosalen und transdermalen Wege gehören.

20. Verwendung gemäß einem beliebigen der Ansprüche 16 bis 19, wobei wie gesagt mindestens eine Metallführung mit einer optischen Faser an mindestens einem Teil der Länge hiervon verbunden ist.
